# DEMANDE DE BREVET EUROPEEN

(11) **EP 0 564 384 A1**
(43) Date de publication de la demande: **06.10.1993**
(21) Numéro de dépôt: 93440031.8
(22) Date de dépôt: 30.03.1993
(51) Int. Cl.: A61B 5/113

(54) **Dispositif d'aide à la surveillance respiratoire**

(30) Priorité: 30.03.1992 FR 9203983
(71) Demandeur: Coral, Hervé, F-27520 Boissey le Chatel (FR); Delattre, Jean-Marie, F-06210 Mandelieu (FR); Hebert, Patrick, F-14540 Garcelles Secqueville (FR)
(72) Inventeur: Coral, Hervé, F-27520 Boissey le Chatel (FR); Delattre, Jean-Marie, F-06210 Mandelieu (FR); Hebert, Patrick, F-14540 Garcelles Secqueville (FR)

(57) **Abrégé**

Dispositif d'aide à la surveillance respiratoire du type comportant une ceinture thoracique inextensible et ajustable en longueur, un capteur de mouvement (2) relié à un dispositif d'alarme et comprenant un boîtier (20) comportant deux attaches permettant la fixation des extrémités de ladite ceinture, l'une des deux attaches étant mobile.

L'attache mobile (21) est mobile en pivotement, par l'une de ses extrémités, autour d'un axe (23) solidaire dudit boîtier (20), et est reliée par son autre extrémité, par l'intermédiaire d'un câble (27), à une extrémité d'une lame conductrice (25) montée pivotante autour d'un axe (26) solidaire dudit boîtier (20), l'autre extrémité (25') de ladite lame (25) étant en appui sur un circuit imprimé (29) comportant une alternance de parties conductrices (29') et de parties isolantes (29''), les parties conductrices (29') et la lame (25) étant reliées électriquement au dispositif d'alarme.

## Description

La présente invention a pour objet un dispositif d'aide à la surveillance respiratoire chez l'homme, particulièrement adapté à la surveillance des nouveau-nés.

Dans le domaine médical et hospitalier, notamment en période post-opératoire, il est parfois nécessaire de surveiller la respiration de patients, afin d'en détecter rapidement les insuffisances ou anomalies.

Dans le cas particulier des nouveau-nés, on sait que ceux-ci sont parfois sujets à des arrêts respiratoires qui sont généralement fatals car non décelés à temps.

Il existe à ce jour des appareils permettant de contrôler et surveiller la respiration d'une personne, toutefois ces appareils sont très complexes et encombrants, et de ce fait ne sont principalement utilisés qu'en milieu hospitalier.

La présente invention vise à proposer un dispositif de surveillance de la respiration, d'une conception simple et d'un encombrement réduit, qui dans le cas notamment de la surveillance d'un nouveau-né, peut être utilisé chez des particuliers dont les enfants présentent des risques d'insuffisance respiratoire.

Le dispositif de surveillance de la respiration selon l'invention comporte une ceinture thoracique inextensible ajustable en longueur, associée à un capteur de mouvement permettant d'enregistrer le rythme ou l'amplitude, des tensions exercées sur ladite ceinture, ces informations étant soit transmises et enregistrées par un boîtier électronique aux fins d'analyse,soit dirigés vers un dispositif programmé pour déclencher une alarme, visuelle ou sonore, si aucune tension n'est enregistrée pendant une certaine durée prédéterminée.

En ce qui concerne la surveillance respiratoire des nouveau-nés, l'alarme peut se déclencher après une période de non variation, donc d'arrêt respiratoire, qui est ajustable, par exemple de 15 à 30 secondes.

Le capteur de mouvement du dispositif selon l'invention comprend un boîtier isolant solidaire de deux attaches permettant la fixation des extrémités de la ceinture thoracique, l'une desdites attaches étant mobile en pivotement par l'une de ses extrémités autour d'un axe solidaire dudit boîtier, tandis que son autre extrémité est reliée, par l'intermédiaire d'un câble de transmission, à une extrémité d'une lame pivotante en matériau conducteur, dont l'autre extrémité balaie en appui, au cours du pivotement, un circuit imprimé comportant une alternance de parties conductrices et de parties isolantes, ledit circuit imprimé étant relié électriquement à un dispositif d'alarme auquel est également reliée ladite lame pivotante.

Les avantages et les caractéristiques de la présente invention ressortiront plus clairement de la description qui suit et qui se rapporte au dessin annexé, lequel en représente un mode de réalisation non limitatif.

Dans le dessin annexé :
- la figure 1 représente une vue schématique partielle, de profil, d'un dispositif selon l'invention.
- la figure 2 représente une vue schématique du capteur de mouvement du dispositif de la figure 1.

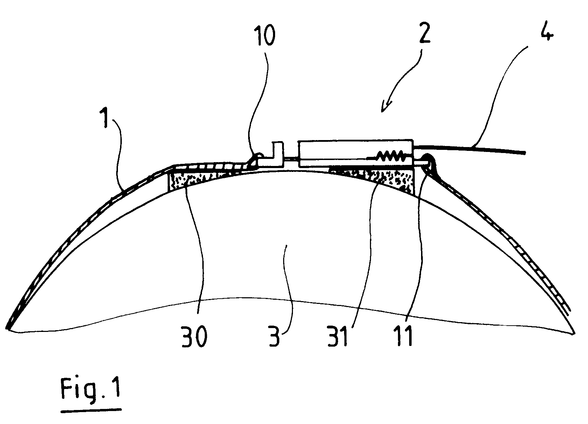

Si on se réfère à la figure 1 on peut voir qu'un dispositif selon l'invention comporte une ceinture 1, inextensible et antidérapante, ajustable par un moyen connu en soi, non représenté, aux deux extrémités 10 et 11 de laquelle est solidarisé un capteur de mouvement 2 relié par l'intermédiaire d'un câble électrique 4 à un dispositif électronique d'alarme, non représenté.

Afin de maintenir le capteur 2 dans une parfaite rectitude, des cales 30 et 31 en matériau semi-rigide sont intercalées entre le capteur 2 et la cage thoracique 3.

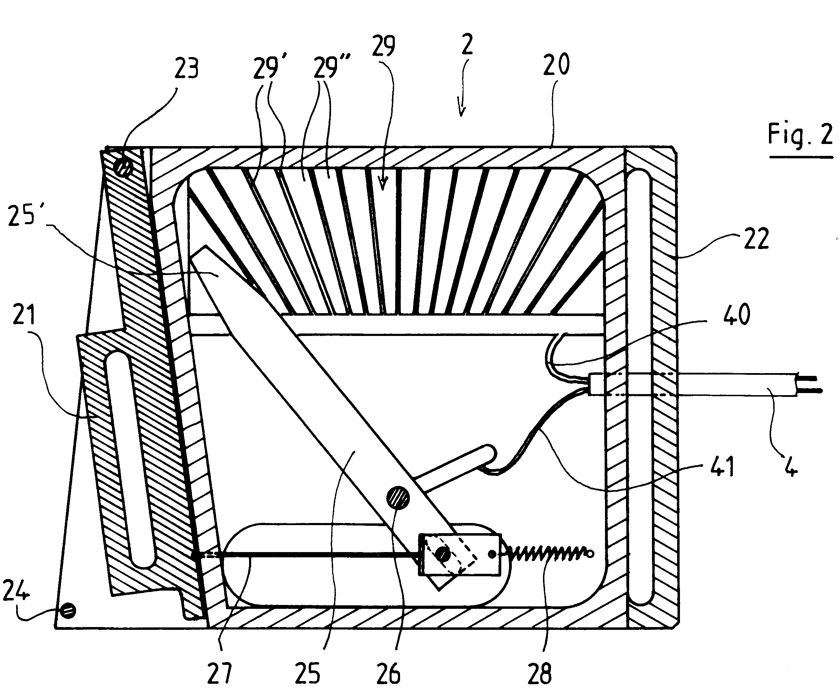

Si on se réfère également à la figure 2 on peut voir que le capteur de mouvement 2 comporte un boîtier isolant 20 comprenant deux attaches 21 et 22 permettant la fixation des extrémités 10 et 11 de la ceinture 1, l'attache 21 étant mobile en pivotement sur le boîtier 20 autour d'un axe 23, une butée 24 solidaire du boîtier 20 limitant ce pivotement.

Une lame 25, réalisée en un matériau conducteur, montée sur un axe 26 solidaire du boîtier 20, peut pivoter autour de cet axe sous l'effet de traction d'un câble de transmission 27 reliant l'une de ses extrémités à l'attache mobile 21. Un ressort 28 solidarisé d'une part au boîtier 20 et d'autre part à la lame 25 en regard du point de fixation du câble 27 permet le rappel de la lame 25.

L'autre extrémité 25' de la lame 25 balaie en appui, au cours du pivotement, un circuit imprimé 29 comportant alternativement des parties conductrices 29' et des parties isolantes 29''.

Le circuit imprimé 29 est relié électriquement, par l'intermédiaire d'un fil 40, à un dispositif électronique d'alarme, non représenté, auquel est également reliée la lame 25 par l'intermédiaire d'un fil 41.

Le dispositif de surveillance selon l'invention est installé sur le patient de la façon suivante: la ceinture 1 est ajustée sur la cage thoracique 3 du patient, à une tension telle que la lame 25 occupe une position médiane sur le circuit imprimé 29. Suivant les mouvements de la cage thoracique 3 l'attache mobile 21 pivote et entraîne le câble de transmission 27 qui lui même provoque le pivotement de la lame 25 et son déplacement sur le circuit imprimé 29, ce qui a pour conséquence d'envoyer vers le dispositif électronique d'alarme une succession de signaux.

L'absence de signaux, au bout d'un temps prédéterminé, a pour effet le déclenchement d'une alarme permettant une intervention immédiate auprès du patient.

Il va de soi que la présente invention ne saurait être limitée à la description qui précède d'un de ses modes de réalisation, susceptible de subir un certain nombre de modifications sans pour autant sortir du cadre de l'invention.

## Revendications

1. Dispositif d'aide à la surveillance respiratoire du type comportant une ceinture thoracique (1) inextensible et ajustable en longueur, un capteur de mouvement (2) relié à un dispositif d'alarme et comprenant un boîtier (20) comportant deux attaches permettant la fixation des extrémités (10, 11) de ladite ceinture (1), l'une des deux attaches étant mobile, caractérisé en ce que l'attache mobile (21) est mobile en pivotement, par l'une de ses extrémités, autour d'un axe (23) solidaire dudit boîtier (20), et est reliée par son autre extrémité, par l'intermédiaire d'un câble (27), à une extrémité d'une lame conductrice (25) montée pivotante autour d'un axe (26) solidaire dudit boîtier (20), l'autre extrémité (25') de ladite lame (25) étant en appui sur un circuit imprimé (29) comportant une alternance de parties conductrices (29') et de parties isolantes (29"), les parties conductrices (29') et la lame (25) étant reliées électriquement au dispositif d'alarme.
